# EUROPEAN PATENT APPLICATION

(11) **EP 4 268 574 A1**
(43) Date of publication of application: **01.11.2023**
(21) Application number: 23169443.1
(22) Date of filing: 24.04.2023
(51) Int. Cl.: A01G 7/00, G01N 33/00, G01N 33/497, G06Q 50/02

(54) **PLANT STATE DETECTION SYSTEM AND GAS DETECTOR**

(30) Priority: 26.04.2022 JP 2022072361
(71) Applicant: Sintokogio, Ltd., Nagoya-shi, Aichi 450-6424 (JP)
(72) Inventor: Mizutani, Manase, Nagoya-shi, Aichi, 450-6424 (JP)
(74) Representative: Hoffmann Eitle

(57) **Abstract**

The plant state detection system (1) includes a gas detector (2) including a gas detection unit (21) configured to detect a gas emitted from a plant, and a server (3) configured to acquire gas information detected by the gas detection unit (21) and detect a state of the plant based on the gas information, wherein the gas detection unit (21) detects one or both of leaf alcohol and leaf aldehyde as the gas, and wherein the IUPAC name of the leaf alcohol is cis-3-hexen-1-ol or trans-2-hexen-1-ol, and the IUPAC name of the leaf aldehyde is trans-2-hexenal or cis-3-hexenal.

## Description

### CROSS-REFERENCE TO RELATED APPLICATION

This application is based on Japanese Patent Application No. 2022-072361 filed with Japan Patent Office on April 26, 2022, the entire contents of which are hereby incorporated by reference.

### TECHNICAL FIELD

The present disclosure relates to a plant state detection system and a gas detector.

### BACKGROUND

Japanese Patent Application Publication No. 2020-64466 discloses, as an apparatus for detecting a plant state, an apparatus that captures an image of a crop and analyzes the captured image to grasp a growth state of the crop or a damage state caused by pests.

### SUMMARY

However, it is difficult for the apparatus described in Japanese Patent Application Publication No. 2020-64466 to accurately detect the plant state. The apparatus described in Japanese Patent Application Publication No. 2020-64466, which captures an image of a plant and detects a plant state, can only detect the state of the plant in a range in which the image is captured, and may fail to detect the state of the plant when a disease, food damage, or the like occurs in a range in which the image is not captured.

The present disclosure provides a plant state detection system and a gas detector capable of accurately detecting a plant state.

That is, a plant state detection system according to one aspect of the present disclosure includes a gas detector including a gas detection unit configured to detect gas emitted from a plant, and a plant state detector configured to acquire gas information detected by the gas detection unit and detect a state of the plant based on the gas information, wherein the gas detection unit detects one or both of leaf alcohol and leaf aldehyde as the gas. According to this plant state detection system, it is possible to detect gas emitted from a plant and detect the state of the plant based on the gas information. Therefore, the plant state detection system can accurately detect the state of the plant. Further, the plant state detection system can detect temperature stress of the plant by detecting one or both of leaf alcohol and leaf aldehyde as the gas.

In the plant state detection system according to one aspect of the present disclosure, the gas detection unit may detect one or both of ethylene and carbon dioxide as the gas. In this case, the plant state detection system can detect the normal growth degree of the plant by detecting one or both of ethylene and carbon dioxide as the gas.

In the plant state detection system according to one aspect of the present disclosure, the gas detection unit may include a semiconductor gas sensor using a metal oxide as a sensor configured to detect leaf alcohol or leaf aldehyde. In this case, by using the semiconductor gas sensor using the metal oxide as the sensor configured to detect leaf alcohol or leaf aldehyde, the plant state detection system can reduce the cost of installation of the gas sensor and can detect various gases.

In addition, the plant state detection system according to one aspect of the present disclosure may include a container configured to accommodate the gas detection unit, and a suction machine configured to suck air around the plant into the container. In this case, the gas detection unit is accommodated in the container, and the gas emitted from the plant can be drawn into the container by sucking air around the plant into the container. Therefore, diffusion of the gas is suppressed, and the plant state detection system can increase the detection accuracy of the gas.

The plant state detection system according to one aspect of the present disclosure, may further include a plurality of gas detectors including the gas detector, and the plurality of gas detectors is arranged at equal intervals in a cultivation area in which plants are cultivated. In this case, the plant state detection system can accurately recognize the state of the plant at each location in the cultivation area by arranging the plurality of gas detectors at equal intervals in the cultivation area.

In addition, the plant state detection system according to one aspect of the present disclosure may include a display control unit configured to display the state of the plant corresponding to an arrangement position of the plurality of gas detectors. In this case, by displaying the state of the plant corresponding to the arrangement position of the plurality of gas detectors, the plant state detection system can allow a user to easily grasp the state of the plant in the cultivation area through the visual sense.

A gas detector according to an aspect of the present disclosure includes a gas detection unit configured to detect gas emitted from a plant, and an output unit configured to output gas information of the gas detected by the gas detection unit as information for detecting a state of the plant, wherein the gas detection unit detects one or both of leaf alcohol and leaf aldehyde as the gas. According to this gas detector, the gas emitted from the plant is detected, and the gas information is output as information for detecting the state of the plant. As a result, the gas detector can detect the state of the plant based on the gas information of the plant, and can accurately detect the state of the plant. The gas detector can detect temperature stress of the plant by detecting one or both of leaf alcohol and leaf aldehyde as the gas.

In the gas detector according to the aspect of the present disclosure, the gas detection unit may detect one or both of ethylene and carbon dioxide as the gas. In this case, the gas detector can detect the normal growth degree of the plant by detecting one or both of ethylene and carbon dioxide as the gas.

In the gas detector according to the aspect of the present disclosure, the gas detection unit may include a semiconductor gas sensor using a metal oxide as a sensor configured to detect leaf alcohol or leaf aldehyde. In this case, by using the semiconductor gas sensor using a metal oxide as the sensor configured to detect leaf alcohol or leaf aldehyde, the gas detector can reduce the cost of installation of the gas sensor and can detect various gases.

In addition, the gas detector according to one aspect of the present disclosure may include a container configured to accommodate the gas detection unit, and a suction machine configured to suck air around the plant into the container. In this case, the gas detection unit is accommodated in the container, and the gas emitted from the plant can be drawn into the container by sucking air around the plant into the container. Therefore, diffusion of the gas is suppressed, and the plant state detection system can increase the detection accuracy of the gas.

In addition, in the gas detector according to one aspect of the present disclosure may include a plurality of gas detection units including the gas detection unit, and the plurality of gas detection units may be arranged at equal intervals in a cultivation area in which plants are cultivated. In this case, by arranging the plurality of gas detection units at equal intervals in the cultivation area, it is possible to accurately recognize the state of the plant at each location in the cultivation area.

According to the present disclosure, it is possible to accurately detect a plant state.

### BRIEF DESCRIPTION OF THE DRAWINGS

FIG. 1 is a diagram showing an outline of an electrical configuration of a plant state detection system and a gas detector according to an embodiment of the present disclosure.
FIG. 2 is a diagram showing an outline of a configuration and an installation mode of the gas detector of FIG. 1.
FIG. 3 is a diagram showing an example of an installation mode of the gas detector shown in FIG. 1.
FIG. 4 is a diagram showing a display mode of a plant state in the plant state detection system of FIG. 1.
FIG. 5 is a flowchart showing a control processing of the gas detector shown in FIG. 1.
FIG. 6 is a flowchart showing a control processing of the server of the plant state detection system shown in FIG. 1.

### DETAILED DESCRIPTION

Hereinafter, embodiments of the present disclosure will be described with reference to the drawings. In the following description, the same or corresponding elements are denoted by the same reference numerals, and redundant description is omitted.

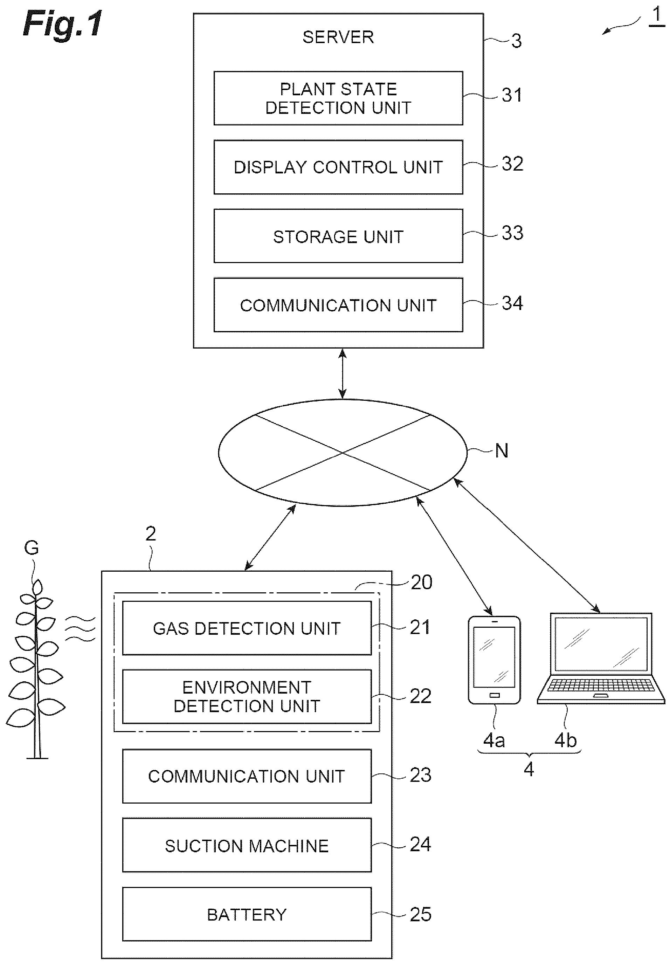

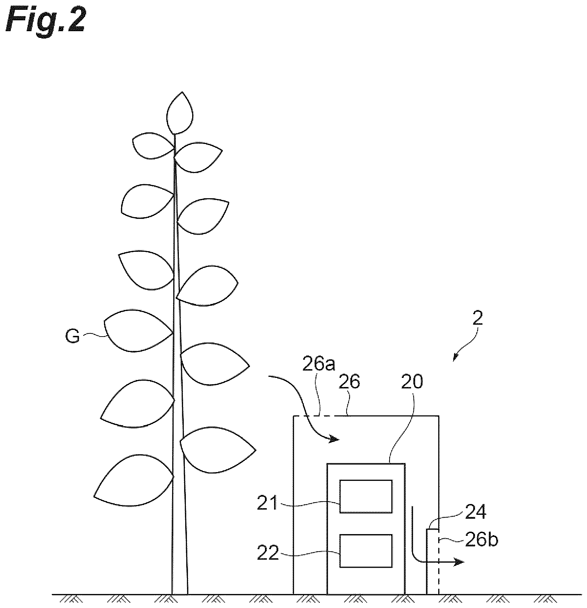

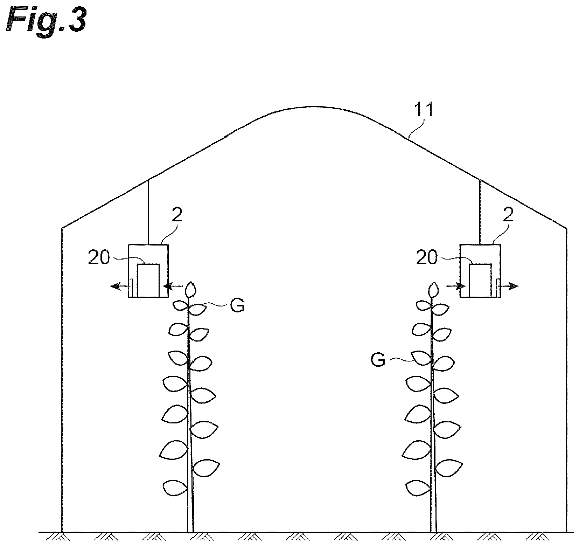

FIG. 1 is a diagram showing an outline of an electrical configuration of a plant state detection system and a gas detector according to an embodiment of the present disclosure. FIGS. 2 and 3 are explanatory views of a configuration mode and an installation mode of the gas detector.

As shown in FIG. 1, a plant state detection system 1 is a system for detecting a state of a plant G by detecting gas emitted from the plant G. The plant state detection system 1 includes a gas detector 2 and a server 3 (an example of a plant state detector).

The gas detector 2 is a device that detects gas emitted from the plant G, and is disposed in the vicinity of the plant G, for example. The plant G is a plant whose state is to be detected, and is, for example, a cultivated plant. The gas detector 2 is configured to transmit data information such as gas information to the server 3. For example, the gas detector 2 and the server 3 are configured to communicate via internet N. The gas detector 2 includes a gas detection unit 21, an environment detection unit 22, a communication unit 23 (an example of an output unit), a suction machine 24, and a battery 25.

The gas detection unit 21 and the environment detection unit 22 are provided as part of a sensor unit 20, for example, arranged in the same housing. The gas detection unit 21 is a gas sensor that detects gas emitted from the plant G. The gas emitted from the plant G includes the gas emitted directly from the plant G, as well as the gas emitted from those attached to the plant G and those attached to the vicinity of the plant G.

The gas to be detected includes, for example, one or both of leaf alcohol (hexenol, IUPAC name of the leaf alcohol is cis-3-hexen-1-ol or trans-2-hexen-1-ol) and leaf aldehyde (hexenal, IUPAC name of the leaf aldehyde is trans-2-hexenal or cis-3-hexena). IUPAC name is defined by International Union of Pure and Applied Chemistry (IUPAC). Leaf alcohol and leaf aldehyde are green leaf volatiles, and are volatile substances having a skeleton of six carbon atoms. Since the gas detection unit 21 detects leaf alcohol or leaf aldehyde as the gas, the temperature stress degree of the plant G can be detected. For example, the plant state detection system 1 may detect that the higher the sensed level (e.g., gas concentration) of leaf alcohol or leaf aldehyde, the greater the temperature stress of the plant G.

Further, the gas to be detected may be one or both of ethylene and carbon dioxide. When the gas detection unit 21 detects ethylene or carbon dioxide as the gas, the normal growth degree of the plant G can be detected. For example, the plant state detection system 1 may detect that the plant G is growing more normally when the sensed level of ethylene or carbon dioxide (e.g., gas concentration) is higher.

The gas detection unit 21 may detect at least one of leaf alcohol, leaf aldehyde, ethylene, and/or carbon dioxide. The gas detection unit 21 may also sense some or all of these gases. Further, the gas detection unit 21 may detect gases other than leaf alcohol, leaf aldehyde, ethylene, and carbon dioxide, which can detect a plant state.

The gas detection unit 21 includes, for example, a metal-oxide semiconductor gas sensor as a sensor configured to detect leaf alcohol or leaf aldehyde. As a result, the gas detector 2 can reduce the cost of the gas detection unit 21 and can detect various gases. The gas detection unit 21 can use a known sensor as a sensor configured to ethylene and carbon dioxide. In the case of detecting a plurality of different gases in the gas detection unit 21, a plurality of sensors may be used as the gas detection unit 21. In addition, a sensor other than the above-described sensors may be used as the gas detection unit 21 as long as the sensor can detect gas.

The environment detection unit 22 is a sensor for sensing the growth environment of the plant G. The environment detection unit 22 detects at least one environmental value among environmental values of temperature, humidity, carbon dioxide concentration, and illumination around the plant G. The environment detection unit 22 may detect a part or all of the environmental values of temperature, humidity, carbon dioxide concentration, and illumination around the plant G. As the environment detection unit 22, for example, a thermometer, a hygrometer, a carbon dioxide concentration meter, or an illuminometer is used. The environment detection unit 22 may be used as a detection sensor for CO₂. The environment detection unit 22 may detect environmental values other than the temperature, humidity, carbon dioxide concentration, and illumination around the plant G.

The sensor unit 20 may include an airflow sensor. The airflow sensor is a sensor that detects the flow of air around the sensor unit 20. The gas detector 2 can detect whether or not the air around the plant G is drawn to the sensor unit 20 side by detecting the flow of air around the sensor unit 20 by the airflow sensor. In some cases, the environment detection unit 22 may be omitted from the sensor unit 20.

The communication unit 23 functions as an output unit that outputs the gas information detected by the gas detection unit 21 as information for detecting the state of the plant G. For example, the communication unit 23 outputs gas information detected by the gas detection unit 21 to an external device. In this case, the communication unit 23 repeatedly outputs the gas information to the external device in a predetermined cycle. This allows the gas detector 2 to provide the state of the plant G in real time. The gas information may include, for example, a sensed level of gas. When the gas detection unit 21 has a plurality of sensors, a plurality of gas information corresponding to the respective sensors are output. In addition to the gas information, the communication unit 23 may output environment value information of the environment value detected by the environment detection unit 22 as information for detecting the state of the plant G. This the communication unit 23 is, for example, a communication device for communicating with the server 3, and is configured to communicate with the server 3 via the internet N. The communication unit 23 may be, for example, a router for connecting the sensor unit 20 to the internet N or a wireless local area network (LAN) router that relays the connection to the router.

The suction machine 24 is a device for drawing air around the plant G to the sensor unit 20. As the suction machine 24, for example, a device capable of sucking air such as a pump or a fan is used. The battery 25 is a capacitor for supplying power to a device that needs power supply in the gas detector 2. Since the gas detector 2 is provided with the battery 25, it is not necessary to connect a power supply cable to the gas detector 2 to draw external power, and installation and handling of the gas detector 2 are facilitated. In some cases, the installation of the battery 25 is omitted in the gas detector 2, and operation is performed by an external power source.

As shown in FIG. 2, the gas detector 2 is placed at the plant G location. For example, the gas detector 2 is placed on soil where the plant G is planted. In this case, the gas detector 2 may be installed on the ground via a member such as a spacer. In addition, as shown in FIG. 3, when the gas detector 2 is placed in a facility 11 such as a plastic greenhouse, the gas detector 2 may be suspended from the top. FIG. 3 is a diagram showing an example of an installation mode of the gas detector shown in FIG. 1.

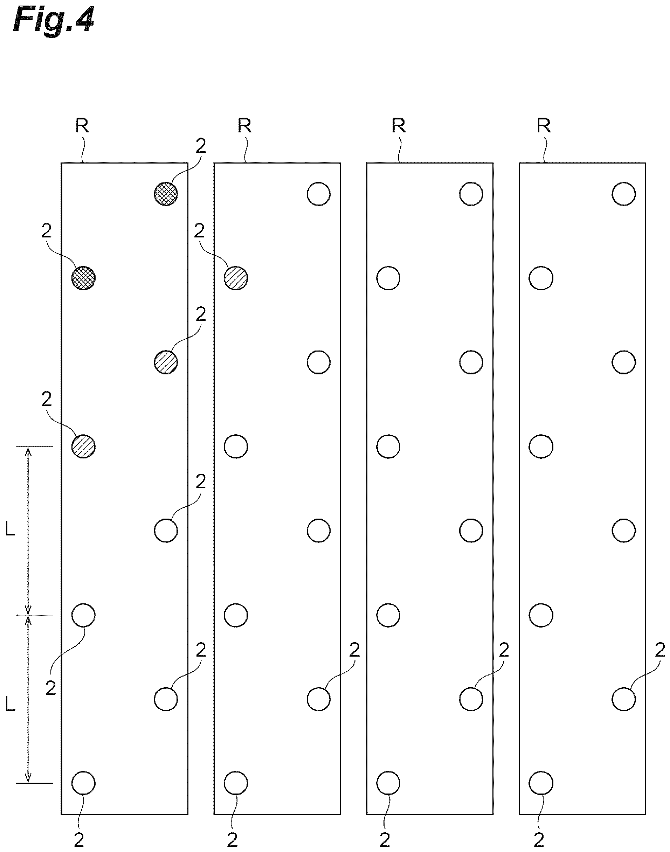

Further, as shown in FIG. 4, a plurality of the gas detector 2 may be installed in a cultivation region R in which a plurality of the plant G is cultivated. FIG. 4 is a view showing an installation mode of the gas detector 2 and a display mode of the plant state, in which the cultivation region R is viewed from above. In this case, the plurality of installed the gas detector 2 is arranged at equal intervals. For example, the gas detector 2 may be placed in the same an interval L for an adjacent the gas detector 2 in the cultivation region R. By arranging the gas detector 2 in this manner, the server 3 can accurately recognize the state of the plant G at each location in the cultivation region R. Although FIG. 4 shows a case where the plurality of gas detectors 2 is arranged at equal intervals in the vertical direction in the cultivation region R, the gas detector 2 may be arranged at equal intervals in the horizontal direction or the oblique direction. Here, the equal interval includes substantially equal interval. The cultivation region R represents a cultivation lot of the plant G, and a plurality of the plant G is cultivated in the cultivation region R.

In FIG. 2, the gas detector 2 has a container 26. The container 26 is a member for accommodating the gas detection unit 21, and for example, a box-shaped member covering the gas detection unit 21 is used. The container 26 may be a rectangular parallelepiped as shown in FIG. 2, or may have a shape other than the rectangular parallelepiped as long as the shape surrounds the gas detection unit 21. As the container 26, for example, one having a size that can be handled by hand is used. In FIG. 2, the sensor unit 20 is contained in the container 26, and the gas detection unit 21 and the environment detection unit 22 are contained in the container 26, however the environment detection unit 22 may be placed outside the container 26. Although the communication unit 23 is not shown in FIG. 2, the communication unit 23 may be installed inside the container 26 or outside the container 26.

A suction port 26a and a discharge port 26b are formed in the container 26. The suction port 26a and the discharge port 26b are openings that pass through the container 26. The suction port 26a is an opening for sucking air around the plant G into the container 26. The suction port 26a is formed, for example, on top of the container 26. The discharge port 26b is an opening for discharging air in the container 26 to the outside of the container 26. The discharge port 26b is formed at a lower portion of the container 26, for example.

The container 26 is provided with the suction machine 24. The suction machine 24 sucks air around the plant G into the container 26. The suction machine 24 is provided, for example, at a position of the discharge port 26b, and draws air around the plant G from the suction port 26a into the container 26 by discharging air in the container 26 from the discharge port 26b by driving. The suction machine 24 may be provided at the position of the suction port 26a, and air may be sucked from the suction port 26a by driving the suction machine 24.

As described above, the container 26 for accommodating the gas detection unit 21 is provided, and the air around the plant G is sucked into the container 26 to detect the gas, whereby the detection accuracy of the gas can be enhanced in the gas detector 2. That is, by sucking the air around the plant G into the container 26, the gas detector 2 can suppress the diffusion of the gas in the plant G and maintain a high concentration of the gas. Thus, the gas detector 2 can improve the detection accuracy of the gas of the plant G.

In FIG. 1, the server 3 is a plant state detector that acquires gas information detected by the gas detection unit 21 and detects the state of the plant G based on the gas information. In addition to the gas information, the server 3 may acquire environmental value information, detect the state of the plant G based on the gas information and the environmental value information, and predict the state of the plant G. The server 3 is configured by a computer including, for example, a central processing unit (CPU), a read only memory (ROM), and a random-access memory (RAM), records gas information and environmental value information transmitted from the gas detector 2, and detects and predicts the state of the plant G based on the information. This the server 3 functions as a main device of cloud computing in the plant state detection system 1, for example. That is, the server 3 provides a terminal device 4 with plant state information obtained by detecting the state of the plant G. For example, the server 3 enables cloud services by providing plant state information to the terminal device 4 that has accessed the server 3.

The server 3 includes a plant state detection unit 31, a display control unit 32, a storage unit 33, and a communication unit 34. The plant state detection unit 31 detects and predicts the plant G conditions based on the gas information and environmental value information. For example, the plant state detection unit 31 obtains the sensed level of the plant G gas as gas information. The sensed level corresponds to the concentration of the gas. The plant state detection unit 31 then determines the state of the plant G based on the sensed level of gas. For example, a table in which the detection level of gas and the state of the plant G are associated with each other, and relationship information such as a determination threshold are set for the plant state detection unit 31. Using this relationship, the plant state detection unit 31 can determine the state of the plant G from the sensed level of gas.

In particular, when the gas is leaf alcohol or leaf aldehyde, the plant state detection unit 31 detects the temperature stress level of the plant G. That is, the plant state detection unit 31 determines whether the plant G is in the normal state, the caution state, or the warning state based on the detected level of leaf alcohol and/or leaf aldehyde. The normal state is a state in which the detection level is low and there is no temperature stress of the plant G, the caution state is a state in which the detection level exceeds the caution level and it is necessary to pay attention to the temperature stress of the plant G, and the warning state is a state in which the detection level exceeds the warning level and it is necessary to take measures against the temperature stress of the plant G. In this case, the plant state detection unit 31 determines the state of the plant G in three stages, but may determine the state in two stages or four or more stages.

When the gas is ethylene or carbon dioxide, the plant state detection unit 31 detects the normal growth degree of the plant G. That is, the plant state detection unit 31 determines whether the plant G is in a normal state, a caution state, or a warning state based on the detected level of ethylene or carbon dioxide. The normal state is a state in which the detection level is high and the plant G grows normally, the caution state is a state in which the detection level exceeds the caution level and the plant G does not grow normally so much, and the warning state is a state in which the detection level exceeds the warning level and the plant G does not grow normally and growth measures are necessary. In this case, the plant state detection unit 31 determines the state of the plant G in three stages, however, may determine the state in two stages or four or more stages.

Further, the plant state detection unit 31 acquires environmental values of temperature, moisture, carbon dioxide concentration, and illumination around the plant G as environmental value information. The plant state detection unit 31 determines the state of the plant G based on the environmental value. For example, in the plant state detection unit 31, a table in which the environmental value and the state of the plant G are associated with each other, a determination threshold value, and the like are set. Using this relationship, the plant state detection unit 31 can determine the state of the plant G from the environmental values. That is, the plant state detection unit 31 can predict the degree of normal growth of the plant G based on the temperature, moisture, carbon dioxide concentration, and illumination around the plant G. In addition, the plant state detection unit 31 can more accurately detect and predict the growth state of the plant G by performing determination using a combination of gas information and environmental value information.

The display control unit 32 indicates the status of the plant G based on the gas sensing level in the gas detector 2. For example, the display control unit 32 generates display date in which the gas detector 2 and the state of the plant G are associated with each other based on the state of the plant G detected by the plant state detection unit 31. Then, the display control unit 32 displays the gas detector 2 and the state of the plant G in association with each other so as to be browsable in the terminal device 4 or the like.

To be specific, as shown in FIG. 4, when a plurality of the gas detection unit 21 are installed in the cultivation region R, the display control unit 32 displays the state of the plant G corresponding to the arrangement position in the gas detector 2. The display mode is changed according to the state of the plant G. For example, the better the state of the plant G, the lighter the display mode. In other words, the display mode is displayed in a darker manner as the state of the plant G is worse. The display control unit 32 can visually and easily grasp the state of the plant G in the cultivation region R by setting different display modes according to the state of the plant G. In addition, the plant G can be efficiently managed in the case of cultivating the plant G in a large-scale cultivation facility, cultivating the plant G in a wide the cultivation region R, cultivating the plant G in a plurality of the cultivation region R, or the like.

The display mode of the state of the plant G may be a different color according to the state of the plant G. For example, when the state of the plant G is good, the color is made inconspicuous. For example, the color is green when the state of the plant G is good, yellow when the state of the plant G is not so good, and red when the state of the plant G is bad. In addition, the display control unit 32 may be displayed with different marks according to the state of the plant G, or the state of the plant G may be displayed with characters as a display mode. In addition, the display control unit 32 may display the state of the plant G in a tabular form by assigning numbers to the gas detection unit 21 as a display mode.

The storage unit 33 records gas information and the plant G condition information. In this case, gas information repeatedly transmitted from the gas detector 2 in a predetermined cycle is recorded, and the state of the plant G based on the gas information is recorded.

The communication unit 34 has the ability to communicate with the gas detector 2. For example, the communication unit 34 may communicate with the gas detector 2 via the internet N and receive gas information transmitted from the gas detector 2. In addition, the communication unit 34 provides information on the state of the plant G detected based on the gas information to the terminal device 4. For example, by accessing the server 3 using the terminal device 4, it is possible to confirm or recognize the status of the plant G through the terminal device 4 even if not at the plant G location. The terminal device 4 is, for example, a smartphone 4a, a personal computer 4b, or the like. The terminal device 4 may also be another terminal device, such as a tablet.

Next, the use method and operation of the plant state detection system 1 and the gas detector 2 according to the present embodiment will be described.

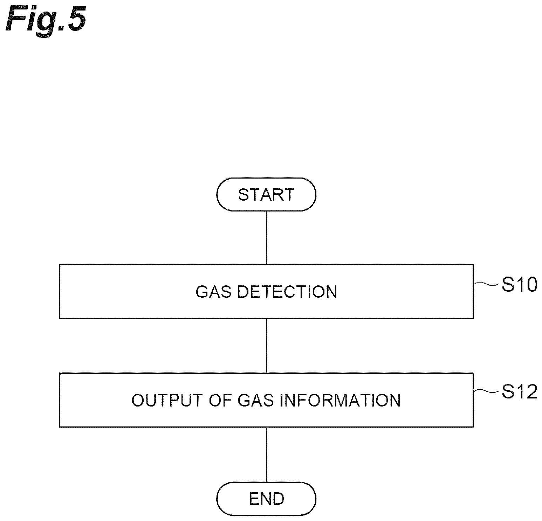

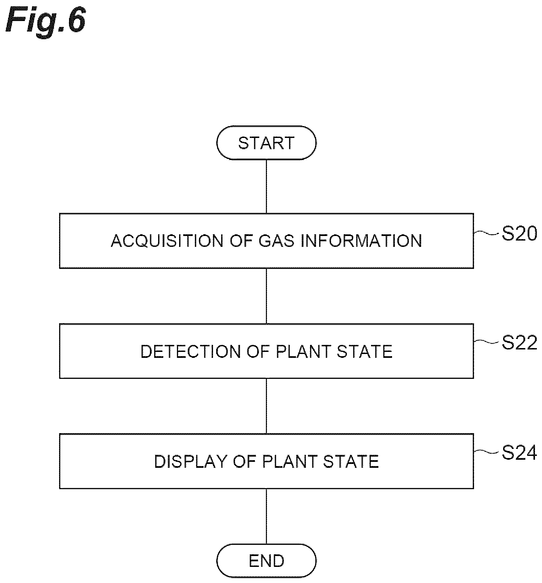

FIG. 5 is a flow chart showing the operation of the gas detector 2. FIG. 6 is a flow chart showing the operation of the server 3 in the plant state detection system 1.

First, as shown in FIGS. 2 and 3, the gas detector 2 is placed in a place where there is the plant G. The gas detector 2 is placed near the plant G. At this time, the gas detector 2 may be placed on the ground or may be lifted from above. Further, as shown in FIG. 4, a plurality of the gas detector 2 may be installed in the cultivation region R of the plant G. In this case, the gas detection units 21 of the gas detector 2 are arranged at equal intervals. Thus, the state of the plant G at each place in the cultivation region R can be accurately recognized. The position of the gas detector 2 is displayed and registered in the cultivation region R. For example, the position of the gas detector 2 in the cultivation region R is set and registered so as to match the position of the display range.

Then, as shown in FIG. 5, the gas detector 2 gas detection process is executed. The series of control processing shown in FIG. 5 is repeatedly executed at a predetermined cycle by the gas detector 2.

First, as shown in step S10 (hereinafter simply referred to as S10, the same applies to the subsequent steps) in FIG. 5, gas detection is performed. Gas detection is a process of detecting gas emitted from the plant G. For example, the gas detection unit 21 senses gas emanating from the plant G. This sensing of gas is sensed by the gas detection unit 21 as a concentration of gas and is data processed and recorded by the gas detector 2 as gas information. In a case where a plurality of sensors is provided in the gas detection unit 21 in order to detect a plurality of gases, respective gas information is recorded.

In addition to gas detection, environmental value detection may be performed in the S 10. The detection of the environmental value is processing for detecting the growth environment of the plant G. For example, the environment detection unit 22 detects at least one environmental values of temperature, humidity, carbon dioxide concentration, and illumination around the plant G. The sensed environmental value is data-processed and recorded by the gas detector 2 as environmental value information.

When gas is detected in the S10, the suction machine 24 is activated. That is, as shown in FIG. 2, the suction machine 24 is activated and air around the plant G is sucked into the container 26. Thus, the gas can be detected with high accuracy. That is, by accommodating the gas detection unit 21 in the container 26 and sucking the air around the plant G into the container 26, the gas is prevented from diffusing around the plant G. Therefore, the gas detection can be performed without lowering the concentration of the gas, and the detection accuracy is improved. In addition, since the air around the plant G is sucked into the container 26, even when the air flows around the plant G in a direction opposite to that of the gas detector 2, the gas can be reliably drawn and detected.

Then, the process proceeds to S12 of FIG. 5, and gas information is output. The output of gas information is the process of transmitting gas information of the gas detected in the gas detection unit 21 to the server 3. For example, the communication unit 23 transmits gas information of the gas sensed by the gas detection unit 21 to the server 3 via the internet N. At this time, when the environmental value of the plant G is detected, the environmental value information is transmitted to the server 3 together with the gas information. When the S12 processing is completed, the series of control processing in FIG. 5 is completed.

On the other hand, in the server 3, plant state detection processing is executed as shown in FIG. 6. This plant state detection processing is repeatedly executed at a predetermined cycle by the server 3.

First, as shown in S20 of FIG. 6, gas information is acquired. The acquisition of gas information is processing for acquiring gas information transmitted from the gas detector 2. For example, the communication unit 34 receives gas information transmitted from the gas detector 2. The received gas information is then recorded in the storage unit 33. When the environmental value information is received together with the gas information, the environmental value information is also recorded in the storage unit 33.

Then, the process proceeds to S22, and the plant state is detected. The detection of the plant state is a process of detecting the state of the plant G based on the gas information. When environmental value information is acquired in addition to the gas information, the state of the plant G may be detected based on the gas information and the environmental value information. For example, the plant state detection unit 31 may detect the state of the plant G based on the gas information of the plant G. That is, the plant state detection unit 31 detects the state of the plant G based on the sensed level of gas.

In detail, the plant state detection unit 31 may detect the temperature stress degree of the plant G when the detected gas is leaf alcohol or leaf aldehyde. Then, the plant state detection unit 31 can detect that the temperature stress degree of the plant G is larger as the detection level of the gas is higher. The plant state detection unit 31 can also detect the normal growth of the plant G when the detected gas is ethylene or CO₂. Then, the plant state detection unit 31 can detect that the normal growth degree of the plant G is larger as the detection level of the gas is higher.

Then, the process proceeds to S24, and the plant state is displayed. In the display of the plant state, display date in which the states of the gas detector 2 and the plant G are associated with each other is generated based on the state of the plant G detected in the S22, and the state of the plant G is displayed in association with the gas detector 2. For example, as shown in FIG. 4, when a plurality of the gas detector 2 is arranged in the cultivation region R, the state of the plant G is displayed corresponding to the arrangement position of the gas detector 2. FIG. 4 schematically shows the cultivation region R, and shows the quality of the state of the plant G in different shades corresponding to the gas detector 2. That is, the better the state of the plant G is, the lighter the display is, and the worse the state of the plant G is, the darker the display is. For example, the lower the temperature stress degree of the plant G is, the lighter the display is, and the higher the temperature stress degree of the plant G is, the darker the display is. In addition, the higher the normal growth degree of the plant G is, the lighter the display is, and the lower the normal growth degree of the plant G is, the darker the display is. In FIG. 4, it can be seen that the state of the plant G at the upper left portion of the four the cultivation region R is bad. By making the display mode different according to the state of the plant G, the plant state detection system 1 can easily grasp the state of the plant G in the cultivation region R through visual observation . In addition, the plant state detection system 1 can efficiently manage the plant G when the plant G is cultivated in a large-scale cultivation facility, when the plant G is cultivated in wide the cultivation region R, or when the plant G is cultivated in multiple the cultivation region R.

The display mode of the state of the plant G may be a different color according to the state of the plant G. For example, when the state of the plant G is good, the color is made inconspicuous. For example, the color is green when the state of the plant G is good, yellow when the state of the plant G is not so good, and red when the state of the plant G is bad.

The state of the plant G shown in FIG. 4 is displayed in the terminal device 4 by accessing the server 3 with the terminal device 4. Accordingly, the user can check the state of the plant G using the terminal device 4. Therefore, even a user at a place remote from the plant G can confirm the state of the plant G in real time. When the S24 processing is completed, the series of control processing in FIG. 6 is completed.

As described above, according to the plant state detection system 1 and the gas detector 2 of the present embodiment, the gas emitted from the plant G is detected, and the gas information is output as information for detecting the state of the plant G. Accordingly, the plant state detection system 1 and the gas detector 2 can detect the state of the plant G based on the gas information of the plant G, and can accurately detect the state of the plant G.

For example, when an image of the plant G is captured and the state of a plant is detected based on the captured image, it is difficult to detect the state of a portion of the plant G that is not captured. In addition, when the state of the plant is detected by the infrared sensor, it is difficult to detect the state of a portion of the plant G that is not within the detection range of the sensor. On the other hand, in the plant state detection system 1 and the gas detector 2 according to the present embodiment, since the state of the plant G is detected based on the gas information of the plant G, it is possible to detect the state of the plant G in a portion distant from the gas detection unit 21 or a portion in the shadow. Therefore, the plant state detection system 1 and the gas detector 2 according to the present embodiment can accurately detect the state of the plant G.

In addition, according to the plant state detection system 1 and the gas detector 2 of the present embodiment, the state of the plant G can be detected based on the environmental value of the plant G in addition to the gas information of the plant G. Therefore, the plant state detection system 1 and the gas detector 2 can more accurately detect and predict the state of the plant based on the gas information and the environmental value information.

Further, according to the plant state detection system 1 and the gas detector 2 of the present embodiment, one or both of leaf alcohol and leaf aldehyde are detected as the gas. Thus, the plant state detection system 1 and the gas detector 2 can accurately detect the temperature stress degree of the plant G.

In addition, according to the plant state detection system 1 and the gas detector 2 of the present embodiment, one or both of ethylene and carbon dioxide are detected as the gas. Thus, the plant state detection system 1 and the gas detector 2 can accurately detect the degree of normal growth of the plant G.

In the plant state detection system 1 and the gas detector 2 according to the present embodiment, a metal-oxide semiconductor gas sensor is used as a sensor for detecting leaf alcohol or leaf aldehyde. Thus, the plant state detection system 1 and the gas detector 2 can reduce the cost of the gas sensor, and can detect various gases.

In addition, according to the plant state detection system 1 and the gas detector 2 of the present embodiment, the gas detection unit 21 can be housed in the container 26, air around the plant G can be sucked into the container 26, and gas emitted from the plant G can be drawn into the container 26. Therefore, the plant state detection system 1 and the gas detector 2 can suppress the diffusion of the gas, and can improve the detection accuracy of the gas.

Further, according to the plant state detection system 1 and the gas detector 2 of the present embodiment, a plurality of the gas detection units 21 is provided in the cultivation region R in which a plurality of the plants G is cultivated, and is installed at equal intervals. Therefore, the plant state detection system 1 and the gas detector 2 can accurately recognize the state of the plant G at each place in the cultivation region R.

In addition, the plant state detection system 1 according to the present embodiment includes the display control unit 32 that displays the state of the plant G corresponding to the arrangement positions of a plurality of the gas detectors 2. Therefore, the plant state detection system 1 can display the state of the plant G corresponding to the arrangement positions of the plurality of the gas detectors 2, and can easily grasp the state of the plant G in the cultivation region R through visual observation.

Although the embodiments of the present disclosure have been described, these embodiments describe some of the embodiments of the plant state detection system and the gas detector according to the present disclosure, and the plant state detection system and the gas detector according to the present disclosure are not limited to those described in the above embodiments. The plant state detection system and the gas detector according to the present disclosure may be obtained by modifying the plant state detection system and the gas detector according to the above-described embodiment or applying the plant state detection system and the gas detector to other components without changing the gist described in each claim.

For example, in the plant state detection system 1 and the gas detector 2 according to the above-described embodiment, gas information detected in the gas detector 2 is transmitted to the server 3 via the internet N. However, gas information may be transmitted via a communication line other than the internet N. In addition, the gas detector 2 may be connected to the plant state detector using a wired or wireless connection, and the gas information detected by the gas detector 2 may be transmitted to the plant state detector. In this case, by causing the personal computer to function as a plant state detector, the personal computer can detect the plant state and display the state of the plant G.

In addition, in the plant state detection system 1 and the gas detector 2 according to the above-described embodiment, the container 26 accommodating the gas detection unit 21 is installed in the vicinity of the plant G. However, the plant G may be accommodated in the container 26 to detect the gas of the plant G and detect the state of the plant G.

In addition, the plurality of the gas detector 2 may be freely installed at irregular intervals. For example, each of the plurality of the gas detector 2 may be installed at a position where gas emitted from the plant G is easily detected. In this case, the server 3 may be configured to be able to acquire the installation positions of the plurality of the gas detector 2. The display control unit 32 of the server 3 can display the state of the gas detector 2 and the plant G in association with each other based on the installation position.

## Claims

1. A plant state detection system comprising:
a gas detector including a gas detection unit configured to detect gas emitted from a plant; and
a plant state detector configured to acquire gas information detected by the gas detection unit and detect a state of the plant based on the gas information,
wherein the gas detection unit detects one or both of leaf alcohol and leaf aldehyde as the gas, IUPAC name of the leaf alcohol is cis-3-hexen-1-ol or trans-2-hexen-1-ol, IUPAC name of the leaf aldehyde is trans-2-hexenal or cis-3-hexenal.

2. The plant state detection system according to claim 1, wherein the gas detection unit detects one or both of ethylene and carbon dioxide as the gas.

3. The plant state detection system according to claim 1, wherein the gas detection unit includes a semiconductor gas sensor using a metal oxide as a sensor configured to detect leaf alcohol or leaf aldehyde.

4. The plant state detection system according to claim 1, further comprising:
a container configured to accommodate the gas detection unit; and
a suction machine configured to suck air around the plant into the container.

5. The plant state detection system according to claim 1, further comprising a plurality of gas detectors including the gas detector, wherein the plurality of gas detectors is arranged at equal intervals in a cultivation area in which plants are cultivated.

6. The plant state detection system according to claim 5, further comprising a display control unit configured to display a state of the plant corresponding to an arrangement position of the plurality of gas detectors.

7. A gas detector comprising:
a gas detection unit configured to detect a gas emitted from a plant; and
an output unit configured to output gas information of the gas detected by the gas detection unit as information for detecting a state of the plant,
wherein the gas detection unit detects one or both of leaf alcohol and leaf aldehyde as the gas.

8. The gas detector according to claim 7, wherein the gas detection unit detects one or both of ethylene and carbon dioxide as the gas.

9. The gas detector according to claim 7, wherein the gas detection unit includes a semiconductor gas sensor using a metal oxide as a sensor configured to detect leaf alcohol or leaf aldehyde.

10. The gas detector according to claim 7, further comprising:
a container configured to accommodate the gas detection unit; and
a suction machine configured to suck air around the plant into the container.

11. The gas detector according to claim 7, further comprising a plurality of gas detection units including the gas detection unit, wherein the plurality of gas detection units is arranged at equal intervals in a cultivation area in which plants are cultivated.
